# EUROPEAN PATENT SPECIFICATION

(11) **EP 3 775 907 B1**
(45) Date of publication and mention of the grant of the patent: **14.12.2022**
(21) Application number: 19713812.6
(22) Date of filing: 29.03.2019
(51) Int. Cl.: G01N 33/574

(54) **DIAGNOSING AND TREATMENT OF A CANCER BASED ON THE OVEREXPRESSION OF THE ADAMTSL5 GENE**
DIAGNOSTIZIERUNG UND BEHANDLUNG VON KREBS BASIEREND AUF DER ÜBEREXPRESSION DES ADAMTSL5-GENS
DIAGNOSTIC ET TRAITEMENT DU CANCER BASÉ SUR LA SUREXPRESSION DU GÈNE ADAMTSL5

(30) Priority: 30.03.2018 EP 18165345
(43) Date of publication of application: 17.02.2021
(73) Proprietor: Universite d'Aix-Marseille (AMU), 13284 Marseille Cedex 07 (FR); Centre National de la Recherche Scientifique - CNRS, 75794 Paris Cedex 16 (FR)
(72) Inventor: MAINA, Flavio, 13260 Cassis (FR); ARECHEDERRA, Maria, 28035 Madrid (ES); DONO, Rosanna, 13260 Cassis (FR); MEAD, Timothy, Cleveland, Ohio 44124 (US); APTE, Suneel, Shaker Heights, Ohio 44122 (US)
(74) Representative: Macquet, Christophe
(86) International application number: PCT/EP2019/058092
(87) International publication number: WO 2019/185919

(56) References cited:
- US-A1- 2009 275 633
- MAHA ABDULLAH ET AL: "ADAMTSL5 and CDH11 : putative epigenetic markers for therapeutic resistance in acute lymphoblastic leukemia", HEMATOLOGY, vol. 22, no. 7, 9 August 2017 (2017-08-09) , pages 386-391, XP055473790, LX ISSN: 1024-5332, DOI: 10.1080/10245332.2017.1299417
- Y. NONOMURA ET AL: "ADAMTSL5 is upregulated in melanoma tissues in patients with idiopathic psoriasis vulgaris induced by nivolumab", JEADV. JOURNAL OF THE EUROPEAN ACADEMY OF DERMATOLOGY AND VENEREOLOGY., vol. 31, no. 2, 1 February 2017 (2017-02-01), pages e100-e101, XP055473873, NL ISSN: 0926-9959, DOI: 10.1111/jdv.13818
- JIATAO LOU ET AL: "Biomarkers for Hepatocellular Carcinoma", BIOMARKERS IN CANCER, 1 January 2017 (2017-01-01), pages 1-9, XP055473853,

## Description

### TECHNICAL FIELD OF THE INVENTION

The invention relates to a method of diagnosis, a pharmaceutical composition, a use of a biomarker, and a method of monitoring treatment as defined in the appended claims.

### BACKGROUND OF THE INVENTION

Cancer is the second leading cause of death in world. Nearly one in six deaths worldwide is due to cancer. For example in 2015, 8.8 million people died of cancer. Therefore, it is important to develop new methods for improving the diagnostic of cancer in patients in need thereof, and notably for predicting their susceptibility to the treatments so as to increase their therapeutic responses, which will results in increased survival expectancies. Some biomarkers for Hepatocellular carcinoma are reviewed by Lou et al. in Biomarker in Cancer, pp. 1-9, DOI: 10.1177/1179299X16684640.

Epigenetics is the study of changes in gene activity, which does not involve the modifications of the DNA sequence and which can be transmitted in cell divisions. Unlike mutations that affect the DNA sequence, epigenetic modifications are reversible. It is of common knowledge that epigenetic abnormalities lead to the development and progression of human diseases, especially cancers. Epigenetic processes intervene in the regulation of many events such as cell division, differentiation, survival, and mobility. The alteration of these mechanisms promotes the transformation of healthy cells into cancer cells, and so any epigenetic aberration may be involved in tumorigenicity. Among consequences linked to epigenetic abnormalities in cells, there is silencing of tumor suppressor genes or overexpression of oncogenes such as proto-oncogenes, which involve aberrant DNA methylation or mutations of genes encoding enzymes responsible for chromatin modifications. DNA methylation is an essential epigenetic mechanism influencing gene expression levels in cells. These alterations may lead to dramatic biological changes as well as acquisition of malignant properties. The cancer landscape is generally characterized by a diffuse DNA hypomethylation and by focal hypermethylation in CpG-rich regions known as CpG islands (CGI). Generally, CGI hypermethylation at promoters represses transcription of genes acting as tumor suppressors. Nevertheless, a large fraction of DNA methylation is also observed in gene body CGIs, with a positive correlation between methylation and upregulation of gene expression. This implies that DNA hypermethylation in gene body CGIs is linked to the upregulations of genes that would act as positive regulators of tumorigenicity, which is coherent with the reported function of some of them in cancer cells. However, the implication of several others hypermethylated and upregulated genes in cell tumorigenicity still remain unknown.

These findings have busted the development of drugs targeting epigenetic regulators, which are called epidrugs or epi-medicines. Two main families of compounds have been notably developed until now: (i) compounds that inhibit the DNA methylation, and (ii) compounds that target the histones modifications. However, at present these types of compounds lack of specific action.

Based on the above, there remains a need for identifying genes that can be used as new specific biomarkers and/or targets to modulate with bioactive agents for cancer treatment. In particular, there remains a need to develop new methods for diagnosis, prognosis, and treatment of cancer.

### SUMMARY OF THE INVENTION

In accordance with a first aspect, the invention relates to a method for diagnosing an hepatocellular carcinoma in a mammal in a need thereof. The method comprises the following steps:
- providing a biological sample collected from said mammal;
- determining, from said biological sample, if the ADAMTSL5 gene is overexpressed;
- diagnosing an hepatocellular carcinoma from the determination of the overexpression of said gene.

According to a second aspect, the invention relates to a pharmaceutical composition. The pharmaceutical composition comprises an agent targeting the ADAMTSL5 gene itself and/or the pathway in which ADAMTSL5 acts, and a pharmaceutically acceptable carrier for use in the treatment of a cancer.

According to a third aspect, the invention concerns an *in vitro* use of an ADAMTSL5 protein as a biomarker of an hepatocellular carcinoma.

In a fourth aspect, the invention relates to an *in vitro* method for monitoring the response to an anticancer treatment of a mammal suffering from hepatocellular carcinoma comprising determining the ADAMTSL5 level of expression in a biological sample of said mammal at two or more time points during said anticancer treatment, wherein an equal or higher ADAMTSL5 level of expression in a biological sample of the subject at a later time point, compared to a reference value obtained in a biological sample of the subject at an earlier time point, is indicative of a resistance of the subject to said anticancer treatment whereas a lower ADAMTSL5 level is indicative of a response of the subject to said anticancer treatment.

Advantageously, the method for diagnosing an hepatocellular carcinoma in a mammal in a need thereof according to the invention is characterized in that: - the biological sample is selected from the group consisting of blood, biopsy tissue, blood serum, blood plasma, urine, stool, sputum, cerebrospinal fluid, or supernatant from cell lysate, preferably the biological sample is tissue biopsy, blood, blood plasma, blood serum, or urine; - the overexpression of ADAMTSL5 gene in the biological sample is determined by measuring the ADAMTSL5 protein levels or mRNA levels in said biological sample; - the ADAMTSL5 protein levels in the biological sample are measured by adding at least one antibody anti-ADAMTSL5 type to said biological sample; - the antibody anti-ADAMTSL5 type is selected from the group consisting of uncoupled or coupled with alkaline phosphatase horse-radish peroxidase, or with fluorescent dyes; - the ADAMTSL5 protein levels in the biological sample are measured by using immunostaining, immunofluorescence, western blot, or ELISA; and - the ADAMTSL5 mRNA levels in the biological sample are measured by available methodologies to detect mRNA to said biological sample, including microarray, RNA-seq, in situ hybridization, RNA-scope, as well as regular, semi-quantitative, or quantitative RT-PCRs.

The pharmaceutical composition according to the invention is characterized in that: - the agents targeting ADAMTSL5 are selected from the group consisting of blocking antibodies, sh-RNA, si-RNA, and antisense RNA against ADAMTSL5.

### BRIEF DESCRIPTION OF DRAWINGS

Other features and aspects of the present invention will be apparent from the following description and the accompanying drawings, in which:
Fig.1 illustrates hypermethylation in the gene body CGI of ADAMTSL5 and overexpression of ADAMTSL5 in a clinically relevant cancer mouse model (*Alb-R26^{Met}* mice);
Fig.2 illustrates overexpression of ADAMTSL5 mRNA and protein in a clinically relevant cancer mouse model (Alb-*R26^{Met}* mice) ;
Fig.3 illustrates the *in vitro* tumorigenic properties of hepatocellular carcinoma (HCC) cells established from a clinically relevant cancer mouse model (*Alb-R26^{Met}* mice) with high expression levels of ADAMTSL5 compared to cells with reduced ADAMTSL5 expression levels;
Fig.4 illustrates the *in vivo* loss of tumorigenic properties of HCC cells following ADAMTSL5 downregulation;
Fig.5 illustrates the *in vivo* acquisition of tumorigenic properties of immortalized hepatocytes from a clinically relevant mouse model (*R26^{Met}* mice) following ADAMTSL5 overexpression;
Fig.6 illustrates ADAMTSL5 expression levels in a clinically relevant cancer mouse model (*Alb-R26^{Met}* mice) at early and latest stages of tumorigenesis;
Fig. 7 illustrates high ADAMTSL5 mRNA levels in 52% of HCC patients, with a predominance in those associated to alcohol taken;

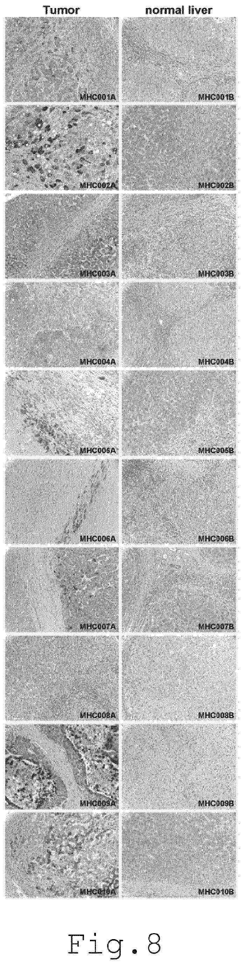

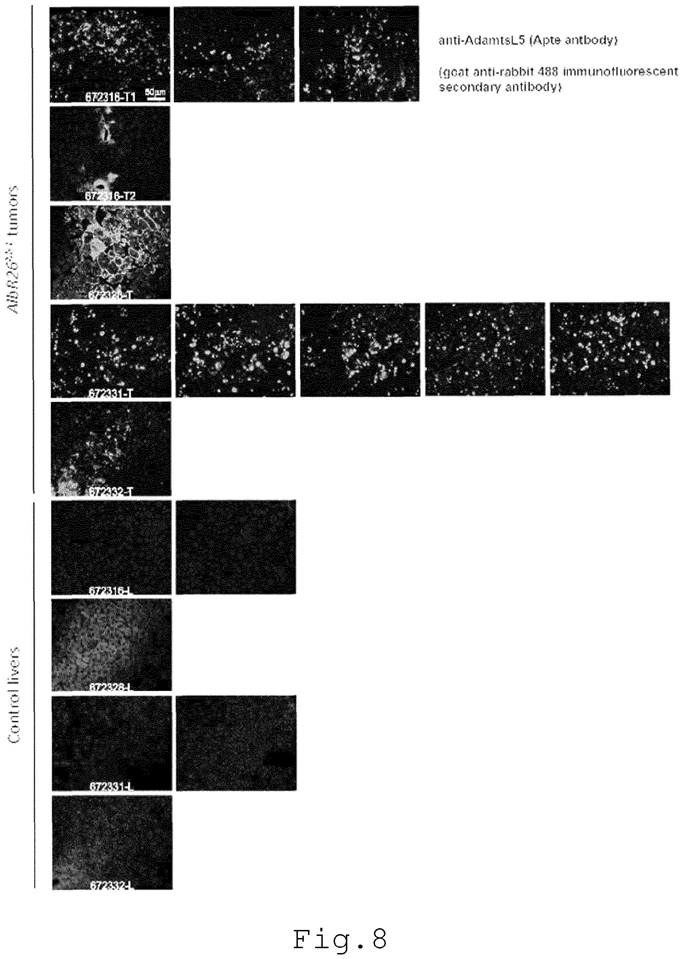

Fig.8 illustrates overexpression of ADAMTSL5 protein levels in 9/10 HCC patients compared to the adjacent liver; and

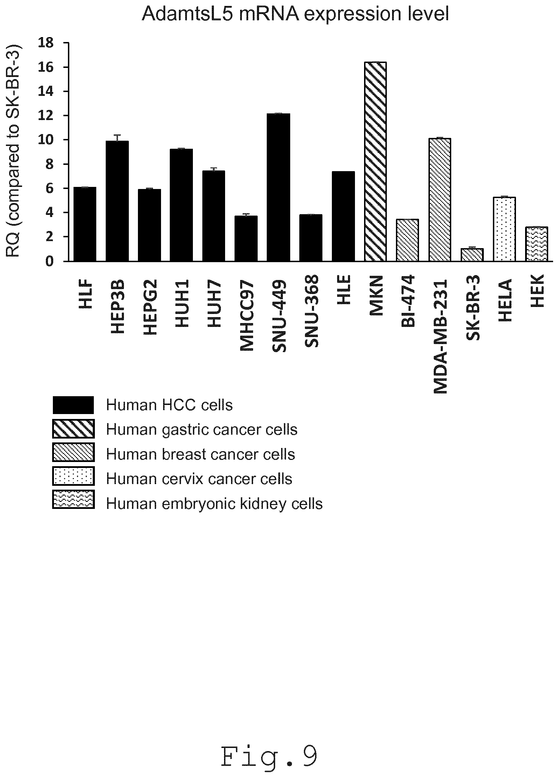

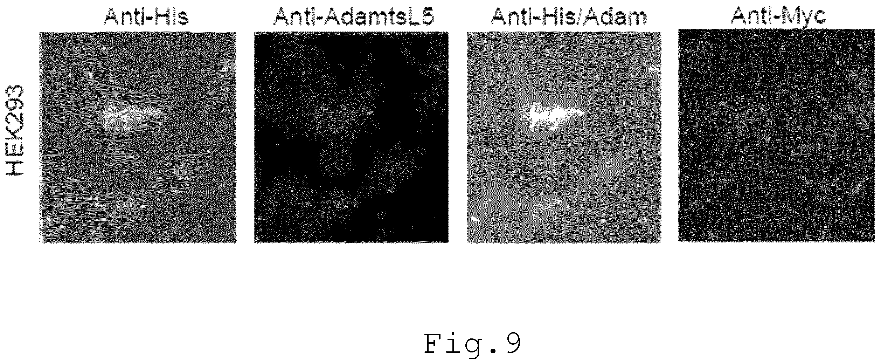

Fig. 9 illustrates ADAMTSL5 mRNA levels in human cancer cell lines.

### DETAILLED DESCRIPTION OF THE INVENTION

The invention relates to a method for diagnosing and prognosis an hepatocellular carcinoma in a mammal in a need thereof. First of all, the method comprises a step of providing an obtained biological sample from said mammal, followed by a step of determining, from said biological sample, if the ADAMTSL5 gene/protein is overexpressed, and then, according to a third step, diagnosing/prognosis a cancer from the determination of the overexpression of said gene or protein.

According to the invention, the mammal is in particular a human. However, all mammals are concerned including even cat, dog, hors or rodents such as mice and rats. According to the invention, the biological sample is selected from the group consisting of blood, tissue biopsy, blood serum, blood plasma, urine, stool, sputum, cerebrospinal fluid, and supernatant from cell lysate. The biological sample that is in particular used is tissue biopsy, blood, blood plasma, blood serum, or urine. According to a preferred embodiment of the invention, the overexpression of ADAMTSL5 gene in the biological sample obtained from the mammals in need thereof is determined by measuring the ADAMTSL5 protein levels or RNA levels in said biological sample. The term "up-regulated", "upregulation", "overexpressed", or "overexpression" is used to mean that the expression, activity, or level of a gene, or RNA transcripts or protein products of the gene, is greater than relative to one or more controls, such as, for example, one or more positive and/or negative controls. In particular, increased levels are considered when levels are higher than those in control healthy tissues.

Practically, mammalian genomes contain 19 ADAMTS (A Disintegrin And Metalloproteinase with ThromboSpondin) genes numbered 1 to 20. Like their relatives, the matrix metalloproteinases (MMPs) and the ADAMs, the ADAMTSs belong to the metzincin protease superfamily, named for the conserved methionine residue close to the zinc iondependent metalloproteinase active site. Representatives of the ADAMTS family are found in all metazoans, although, to date, they have not been identified in single-cell organisms or in plants. All ADAMTSs are secreted, extracellular enzymes that have a compound domain organization, comprising, from the amino-terminus: a signal peptide followed by a pro-region of variable length; a metalloproteinase domain; a disintegrin-like domain; a central thrombospondin type 1 sequence repeat (TSR) motif; and a cysteine-rich domain followed by a spacer region. Separate from the ADAMTSs, another family of seven ADAMTSlike genes (*ADAMTSL*) encode proteins that resemble the ancillary domains of ADAMTS, although lack their catalytic domains. These ADAMTSL proteins, which include ADAMTSL 1 to 6 and papilin, may function to modulate the activities of the ADAMTSs. ADAMTSL5 is a protein that has been discovered in the late 2000s and is described to bind to fibrillin-1 and to promote fibril formation. The role of ADAMTSL5 in microfibril formation is of considerable interest as a crucial mechanism for growth factor regulation in extracellular matrix. ADAMTSL5 is more particularly a secreted protein with a unique domain composition, comprising an N-terminal thrombospondin type 1 repeat, a cystein-rich module, a spacer module, and a C-terminal netrin-like module, which is connected to the spacer by a proline-rich segment. ADAMTSL5 is known as already involved in some disease such as psoriasis but, to date, ADAMTSL5 has not been linked to cancer, as potential biomarker or as a target for molecular therapies.

In the context of the present invention and in a preferred embodiment, the ADAMTSL5 protein level is measured by adding at least one antibody to said biological sample. The antibody, which is in particular used for measuring the ADAMTSL5 protein levels, is of an anti-ADAMTSL5 type. The antibody anti-ADAMTSL5 is in particular selected from the group consisting of uncoupled or coupled or conjugated with alkaline phosphatase, horseradish peroxidase (HRP), or with fluorescent dyes.

According to the invention, the ADAMTSL5 protein levels present in the biological sample of the mammals in need thereof is in particular measured by using immunostaining, immunofluorescence, western blot, or ELISA.

In biochemistry, immunostaining is known as use of an antibody-based method to detect a specific protein in a biological sample. Immunostaining encompasses a broad range of techniques used in histology, cell biology, and molecular biology, which use antibody-based staining methods. Immunofluorescence is a technique used for light microscopy with a fluorescence microscope and is used primarily on microbiological samples. This technique uses the specificity of antibodies to their antigen to target fluorescent dyes to specific biomolecule targets within a cell, and therefore allows visualization of the distribution of the target molecule through the sample.

A kit for determining an overexpression of the ADAMTSL5 gene in a biological sample obtained from a mammal is also described. The kit comprises at least one antibody anti-ADAMTSL5 type and a container for holding the biological sample. The antibodies anti-ADAMTSL5 type used in the kit are the same as those above-described.

According to the invention, ADAMTSL5 levels present in the biological sample of the mammals in need thereof can be also determined by measuring ADAMTSL5 mRNA levels using, for example, microarray, RNA-seq, in situ hybridization, RNA-scope, as well as regular, semi-quantitative, or quantitative RT-PCRs.

According to a second aspect, the invention relates to a pharmaceutical composition. The pharmaceutical composition **comprises** an agent as defined in independent claim 8. In an unclaimed aspect, a demethylating agent is also described, such as for example Decitabine, or agents modulating glycosylation and/or heparin binding. A demethylating agent, according to this description, is a compound that leads to genomic DNA hypomethylation by inhibiting the DNA methyltransferase.

In a third aspect, the invention concerns an *in vitro* use of an ADAMTSL5 protein as a biomarker of an hepatocellular carcinoma.

In a fourth aspect, the invention relates to an *in vitro* method for monitoring the response to an anticancer treatment of a mammal suffering from cancer comprising determining the ADAMTSL5 level of expression in a biological sample of said mammal at two or more time points during said anticancer treatment, wherein an equal or higher ADAMTSL5 level of expression in a biological sample of the subject at a later time point, compared to a reference value obtained in a biological sample of the subject at an earlier time point, is indicative of a resistance of the subject to said anticancer treatment whereas a lower ADAMTSL5 level is indicative of a response of the subject to said anticancer treatment.

### EXAMPLES: MATERIALS AND METHODS:

In the following examples, genetically modified mice where used. In these mice, the expression levels of Met, a receptor tyrosine kinase (RTK) activated in about 50% of human HCCs, is slightly enhanced above the endogenous level, based on a genetic approach allowing modulation of gene expression in a tissue/temporal-specific manner *(R26^{stopMet}* mice; Fan et al. PLoS Genetics 2015; Fan et al. Hepatology 2017). It was recently demonstrated that enhanced methylation (Met) RTK expression levels in the liver perturbs tissue homeostasis, leading to tumor initiation and evolution into HCC *(Alb-R26^{Met}).* Based on comparisons of Met expression levels in *Alb-R26^{Met}* (n=32) and human (n=249) liver tumors, it was shown that Met levels in the *Alb-R26^{Met}* genetic setting (3.16±0.06 versus control livers) correspond to those found in about 20% of HCC patients (48/249). By analyzing 96 different genes in a panel of tumor samples (n=32), it was shown that liver tumorigenesis modelled by the *Alb-R26^{Met}* mice corresponds to a subset of HCC patients, thus establishing the clinical relevance of the *Alb-R26^{Met}* HCC mouse model. It was used the *Alb-R26^{Met}* mouse model to explore the impact of DNA methylation on transcriptional switches associated with tumorigenesis. It was identified a striking enrichment in genes simultaneously hypermethylated in CpG islands (CGIs) and overexpressed. Among them, it is found ADAMTSL5.

Example 1: Hypermethylation in the gene body CGI of ADAMTSL5 and overexpression of mRNA ADAMTSL5 in the Alb-*R26^{Met}* tumor model compared with control livers.

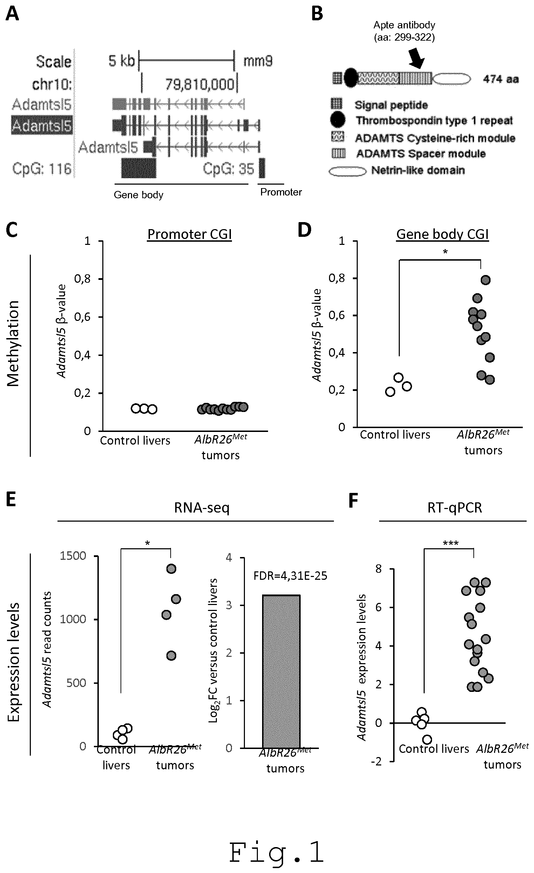

Fig.1(A) shows a schematic representation of mouse ADAMTSL5 transcripts and CGIs from the UCSC genome browser, and Refseq gene annotations based on the NCBI37/mm9 mouse reference. The scheme allows visualization of exons, introns and CGIs of *AdamtsL5* , highlighting with a square the gene body CGI found hypermethylated in tumors.

Fig.1(B) is a schematic representation of the human ADAMTSL5 protein from Badel et al. Matric Biology, 2012. The different domains present in the ADAMTSL5 protein, which could be involved in the modulation of ADAMTSL5 interactions with other protein and in ADAMTSL5 biological functions, are reported.

Fig.1(C) and (D) illustrate the methylation levels (β-value) of *ADAMTSL5* in the promoter CGI (Fig.1(C)) and in the gene body CGI (Fig.1(D)) in control livers (n=3) and *Alb-R26^{Met}* tumors (n=10). It is noted that the methylation levels of *ADAMTSL5* in the promoter CGI is similar in control and tumor samples, whereas the methylation levels in gene body CGI are significantly higher in *Alb-R26^{Met}* tumors compared to control livers. It is concluded that tumors are characterized by a hypermethylation of *ADAMTSL5* in the gene body CGI.

Fig.1(E) illustrates the *ADAMTSL5* mRNA expression levels in *Alb-R26^{Met}* tumors compared to control livers by RNA-seq. The graphs show the read counts (on the right) and Log₂FC (fold-change)(on the left). Significance: *: P<0.05; FDR (p-value adjusted) = 4.31^{E-15}. It is concluded that tumors are characterized by an overexpression of *ADAMTSL5* mRNA.

Fig.1(F) illustrates the *ADAMTSL5* expression levels (by RT-qPCR) in *Alb-R26^{Met}* tumors (n=16) relative to control livers (n=5). It is noted high expression levels of *ADAMTSL5* in *Alb-R26^{Met}* tumors compared to control livers. It is concluded that tumors are characterized by a consistent overexpression of *ADAMTSL5* mRNA.

Example 2: Overexpression of ADAMTSL5 mRNA and protein in a *Alb-R26^{Met}* tumor model.

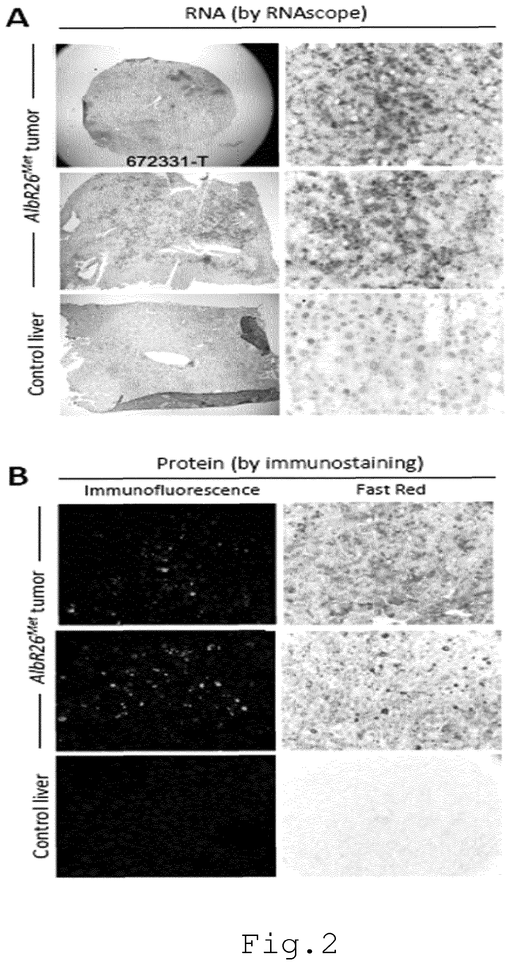

Fig.2(A) shows representative images of the *ADAMTSL5* mRNA expression levels (by RNA-scope) in tumors (dissected from *Alb-R26^{Met}* mice) and in control liver (dissected from control mice). Strong staining is observed in *Alb-R26^{Met}* tumors, but not in control livers. It is concluded that, similar to data from RNA-seq and RT-qPCR analyses, tumors are characterized by a consistent overexpression of *ADAMTSL5* mRNA.

Fig.2(B) shows representative images of the ADAMTSL5 protein levels by immunofluorescence (left) and immunostaining (right) in tumors (dissected from *Alb-R26^{Met}* mice) and in control liver (dissected from control mice). It is noted that ADAMTSL5 is overexpressed in *Alb-R26^{Met}* tumors compared to control livers. It is concluded that, tumors are characterized by a consistent overexpression of ADAMTSL5 protein, coherent with high mRNA levels.

Example 3: *In vitro* tumorigenic properties of *Alb-R26^{Met}* HCC cells, in which ADAMTSL5 is overexpressed. *: P<0.05; **: P<0.01; ***: P<0.001.

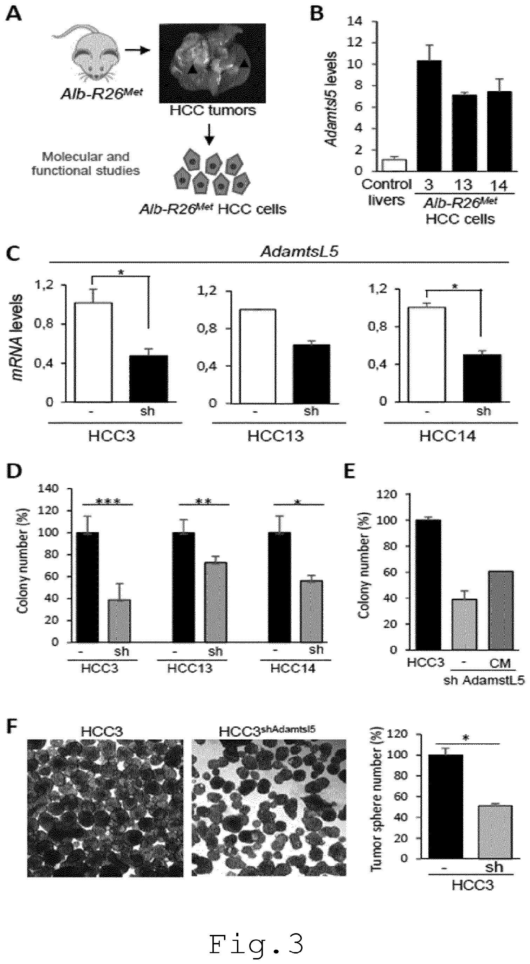

Fig.3(A) is a schematic representation of the establishment of *Alb-R26^{Met}* HCC cell lines (Fan et al. Hepatology, 2017) used for molecular and functional studies. The scheme recapitulates how HCC cell lines have been generated for their molecular characterization and for their use in biological assays. Following the protocol, it is established and reported in Fan et al. Hepatology, 2017, that HCC cell lines were generated from liver tumors dissected from different *Alb-R26^{Met}* mice. Briefly, dissected tumors were minced, incubated in a solution containing collagenase and DNAse, and tissue debris were removed using a 100pm sterile filter. Cells were then cultured in William E medium with supplements either directly in adherent conditions or for a week in non-adherent conditions to enrich cells with tumorigenic properties. After expansion of cultured cells, a proportion of them was frozen and kept as stocks, whereas another proportion was characterized molecularly (biochemistry, RT-qPCR) and functionally (for their capacity to form tumors in nude mice).

Fig.3(B) is a graph reporting the *ADAMTSL5* expression levels (by RT-qPCR) in three *Alb-R26^{Met}* HCC cell lines (HCC3, HCC13, and HCC14) relative to control livers. It is concluded that HCC cells are characterized by a consistent overexpression of *ADAMTSL5* mRNA, as shown in tumors.

Fig.3(C) shows the mRNA expression levels of *ADAMTSL5* in *Alb-R26^{Met}* HCC cells stably transfected with a plasmid carrying a shRNA targeting sequence (carrying also the puromycin gene for selection of stable clones) versus controls. Cells are transfected with the plasmid of interest, then exposed after 48 hours to puromycin for 7 days in order to select cells in which the plasmid has been stably integrated. Clones in which the expression levels of ADAMTSL5 are downregulated by the shRNA targeting sequence are identified by RT-qPCR. It is concluded that the shRNA targeting sequence against ADAMTSL5 leads to an efficient downregulation of *ADAMTSL5* mRNA levels in HCC cells, which interfere with ADAMTSL5 protein expression levels.

Fig.3(D) illustrates results of anchorage independent growth assay (soft agar assay) performed using either HCC control cells or HCC cells carrying the shRNA sequence targeting ADAMTSL5. This assay exemplifies the capacity of cells to form colonies in non-adherent condition, therefore revealing their *in vitro* tumorigenic properties.

Results show that the number of colonies formed by the *Alb-R26^{Met}* HCC cells carrying a shRNA sequence targeting ADAMTSL5 is reduced compared with control cells. It is concluded that high levels of ADAMTSL5 in HCC cells is required for their *in vitro* tumorigenic properties.

Fig.3(E) is descriptive of the anchorage independent growth assay (soft agar assay) showing partially rescue of *in vitro* tumorigenic properties of ADAMTSL5-targeted Alb-*R26^{Met}* HCC cells with condition media from control cells. The results indicate that extracellular ADAMTSL5 confers tumorigenicity to cells expressing low levels of ADAMTSL5. It is concluded that ADAMTSL5, which is a secreted protein, elicits its function in the extracellular environment and can act as well in a cell non-autonomous manner.

Fig.3(F) are representative images (left) and quantification (right) of tumor spheres derived from control and ADAMTSL5-targted *Alb-R26^{Met}* HCC cells (right). It is noted higher numbers and size of tumor sphere generated from control cells compared with *Alb-R26^{Met}* HCC^{shAdamtsl5} cells (carrying the shRNA sequence targeting ADAMTSL5). It is conclude that high levels of ADAMTSL5 in HCC cells confers self-renewal capabilities.

Example 4: Loss of *in vivo* tumorigenic properties of HCC cells following ADAMTSL5 downregulation in *Alb-R26^{Met}* HCC cells. *: P<0.05; **: P<0.01; ***: P<0.001.

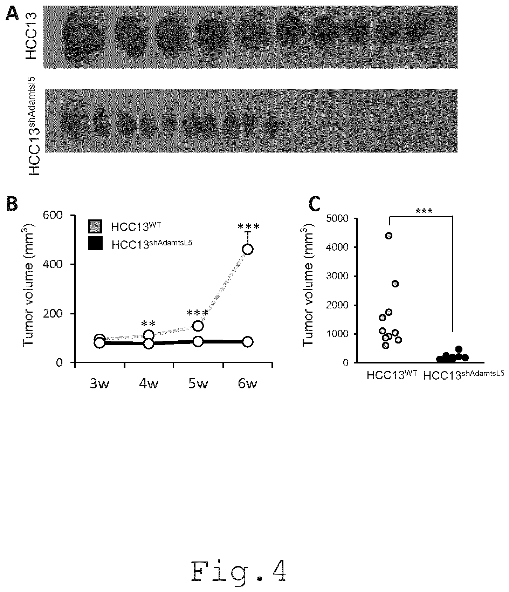

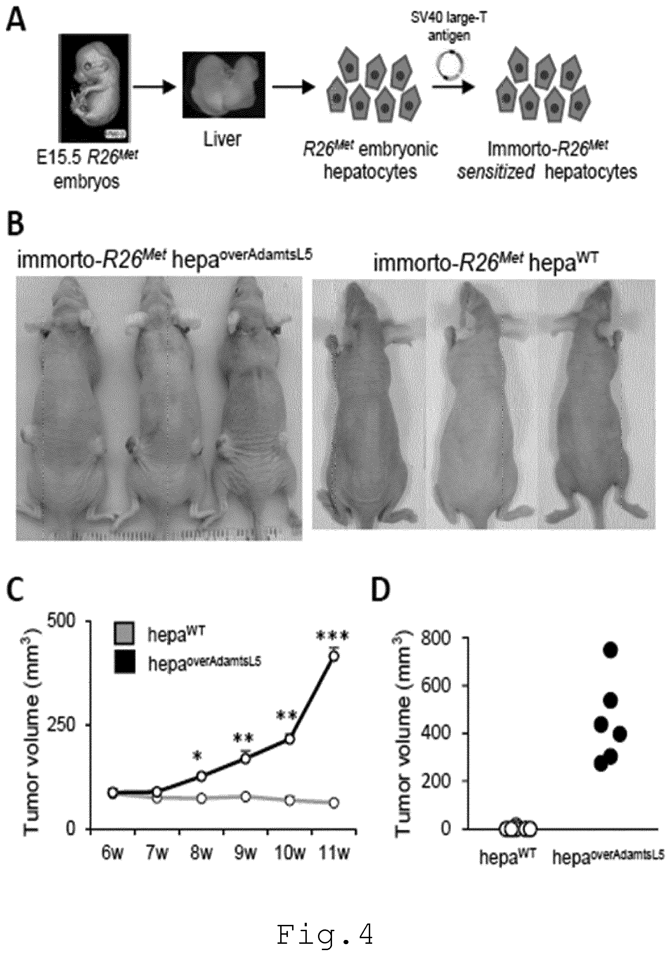

Fig.4(A) contains images of dissected tumors from xenografts in nude mice injected either with *Alb-R26^{Met}* HCC cells (top) or with *Alb-R26^{Me}*-shADAMTSL5 HCC cells (bottom).

Xenograft studies were performed by subcutaneous injection of *Alb-R26^{Met}* or *Alb-R26^{Met}-*shAdamtsL5 HCC cells (5×10⁶ cells) in the flank of nude mice. Fig.4(A) comprises images of dissected tumors after 8 weeks of xenografts establishment. It is noted that tumors are formed in nude mice wherein the *Alb-R26^{Met}* HCC cells were subcutaneous injected, whereas tumors from mice wherein *Alb-R26^{Met}*-shADAMTSL5 HCC cells were subcutaneous injected are drastically reduced, illustrating impaired cell tumorigenic properties *in vivo.*

Fig.4(B) contains xenograft growth curves reporting the mean tumor volume per group measured every week. It is concluded that downregulation of ADAMTSL5 levels in HCC cells (achieved by stable transfection of a shRNA targeting sequence) interferes with *in vivo* tumorigenic properties of HCC cells. Collectively, these results show that downregulation of ADAMTSL5 expression levels interfere with tumor establishment/evolution.

In Fig.4(C), it is shown a quantitative analysis of the volume of tumors dissected 8 weeks after cell injection. Each dot corresponds to the volume of each tumor. It is concluded that downregulation of AdamtsL5 levels in HCC cells (achieved by stable transfection of a shRNA targeting sequence) interferes with *in vivo* tumorigenic properties.

Example 5: Acquisition of in vivo tumorigenic properties following ADAMTSL5 overexpression by immorto-*R26^{Met}* sensitized hepatocytes.

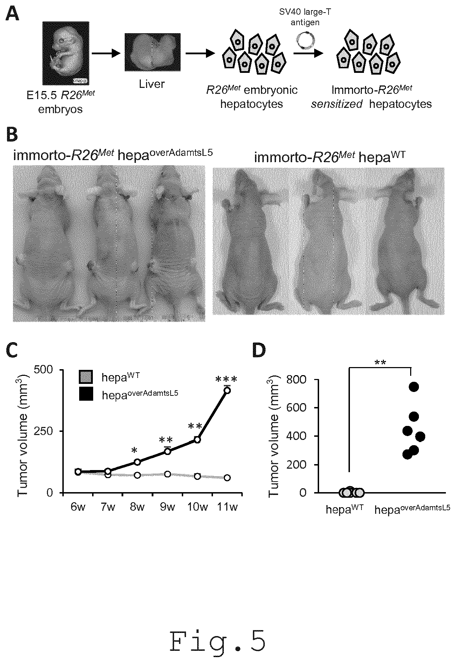

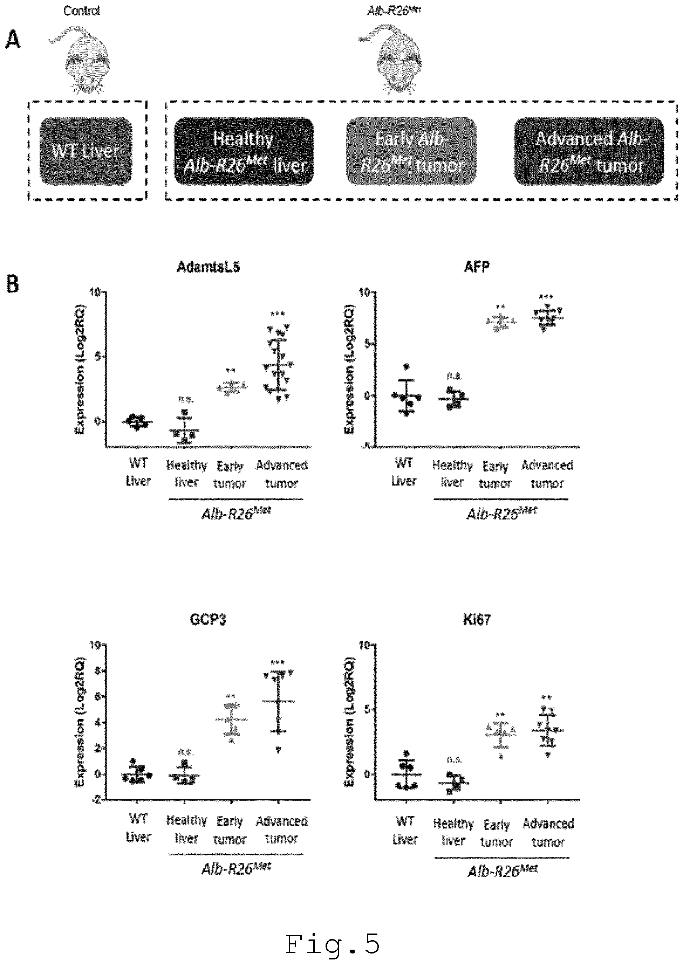

Fig.5(A) is a schematic representation of the establishment of immorto-*R26^{Met}* sensitized hepatocytes (embryonic hepatocytes carrying increased levels of the Met RTK and immortalized with the SV40 large-T antigen). Briefly, cultured hepatocytes from E15.5 mouse *R26^{Met}* embryonic livers were infected with a retrovirus carrying the SV40 large-T antigen for immortalization (plus the neomycin gene for selection of stable clones), then subsequently treated for 7 days with a media permissive for hepatocytes to deplete other cell types. The immorto-*R26^{Met}* hepatocytes are sensitized because of 3-folds increased of wild-type Met levels, although not tumorigenic as incompetent to form tumors in xenografts.

Fig.5(B) comprises images of mice after 11 weeks of xenografts establishment. It is noted that nude mice wherein subcutaneous injection of immorto-*R26^{Met}* hepa^{overAdamtsl5} (cells overexpressing ADAMTSL5) develop tumors in both flanks.

Xenograft studies were performed by subcutaneous injection of immorto-*R26^{Met}* control (immorto-*R26^{Met}* hepa^{WT}) or overexpressing ADAMTSL5 (immorto-*R26^{Met}* hepa^{overAdamtsl5}) hepatocytes (5×10⁶ cells) in both flanks of nude mice.

Fig.5(C) contains xenograft growth curves reporting the mean tumor volume per group measured every week.

In Fig.5(D), it is shown a quantitative analysis of the volume of tumors dissected 11 weeks after cell injection. Each dot corresponds to the volume of each tumor. It is noted that all mice injected with immorto-*R26^{Met}* hepa^{overAdamtsl5} formed tumors in contrast to mice injected with immorto-*R26^{Met}* hepa^{WT}. It is concluded that overexpression of ADAMTSL5 levels in immorto-hepatocytes, which are not tumorigenic as incompetent to form tumors in xenografts, is sufficient to confer to them *in vivo* cell tumorigenic properties.

Example 6: ADAMTSL5 expression level in *Alb-R26^{Met}* tumors at early and latest stages.

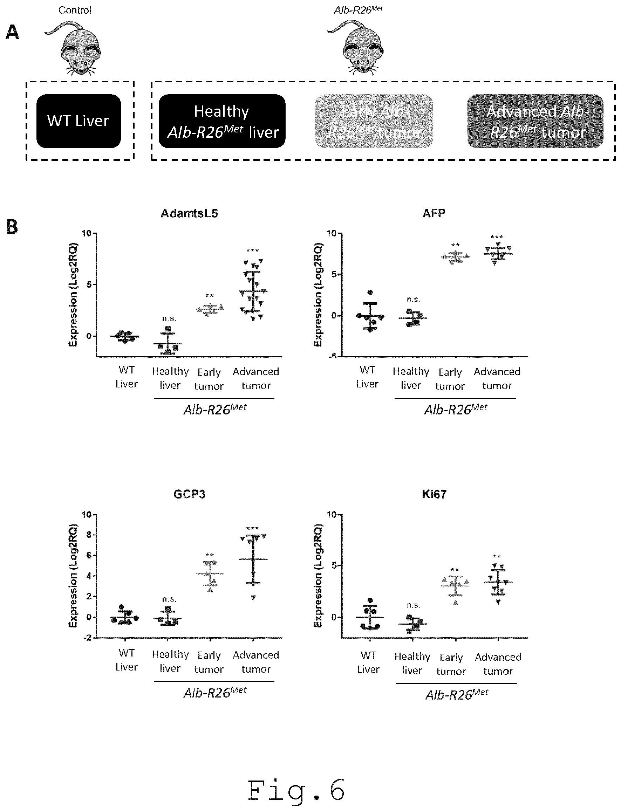

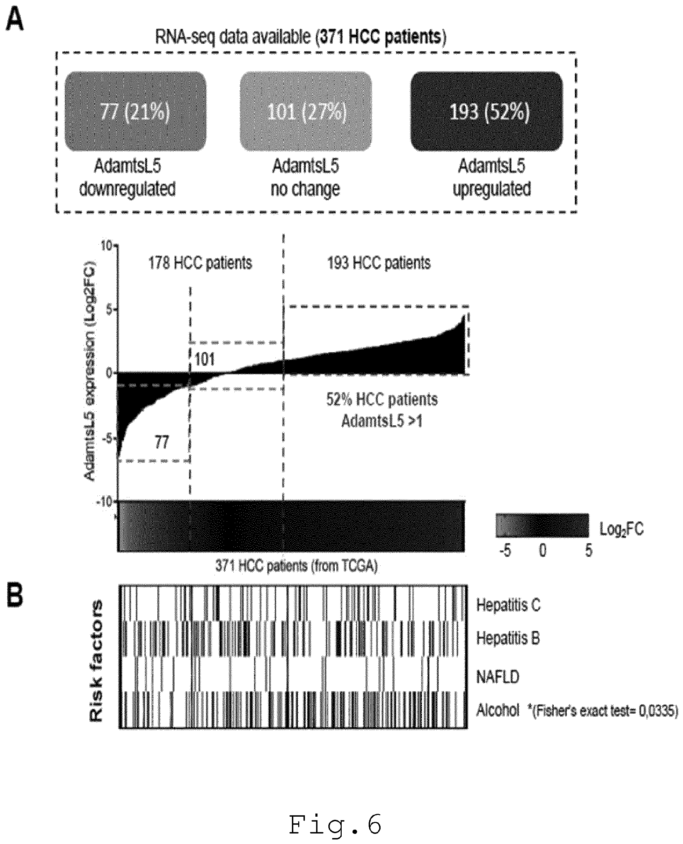

Fig.6(A) is a schematic representation of the tissue samples used for RT-qPCR analysis of ADAMTSL5 levels. The samples used are: control wild-type livers (WT), *Alb-R26^{Met}* healthy livers, *Alb-R26^{Met}* tumors at early and advanced stages.

In Fig.6(B), the graphs report the expression levels (by RT-qPCR) of *ADAMTSL5, AFP* (alpha-fetoprotein), *GPC3* (Glypican-3) (two HCC markers), and *Ki67* (a proliferative marker). It is noted comparable low expression levels of *ADAMTSL5* as well as for the other analyzed markers in wild-type and *Alb-R26^{Met}* healthy livers. In contrast, transcript levels are already increased in *Alb-R26^{Met}* tumors at early stages. It is concluded that *ADAMTSL5* transcript levels can be used to discriminate healthy from neoplastic samples already at early tumorigenic state.

Example 7: High *ADAMTSL5* mRNA levels are present in 52% of HCC patients, with a predominance in those associated to alcohol taken.

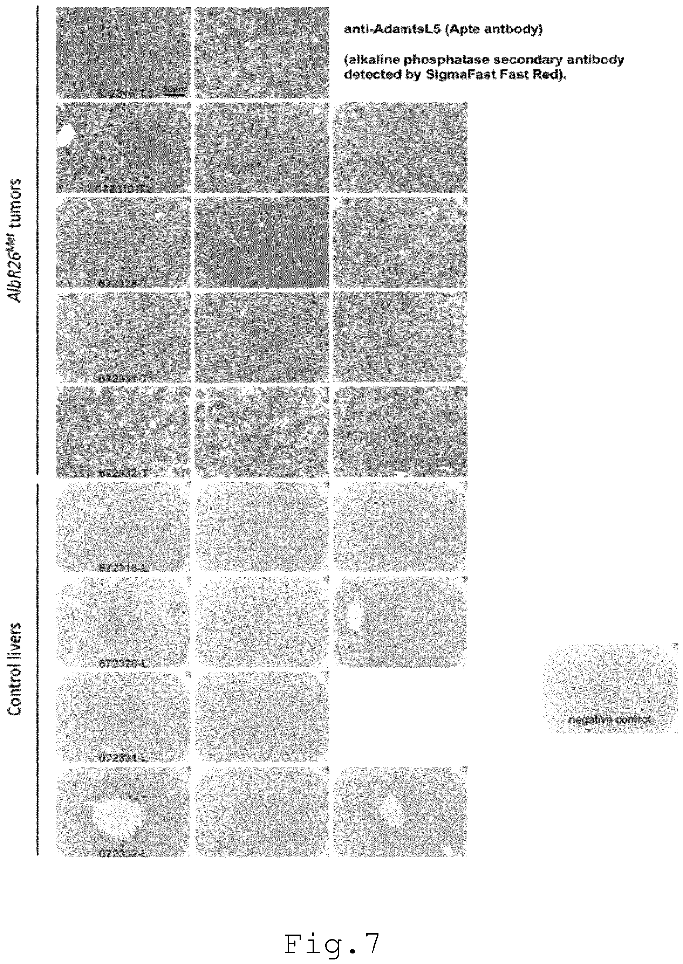

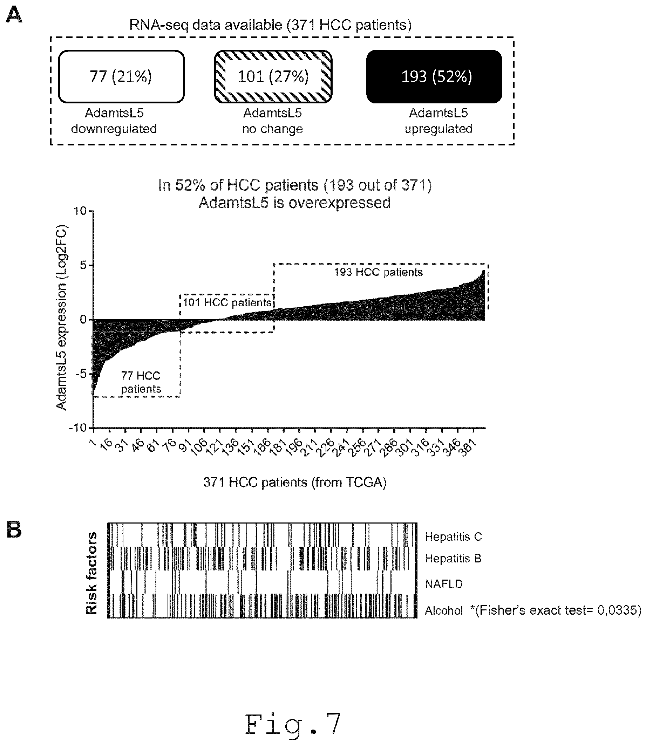

In Fig.7(A), the cohort of HCC patients (371 patients) with *ADAMTSL5* mRNA levels is reported on the top: 21% are characterized by *ADAMTSL5* downregulation (white, left), 27% by no changes (white with black lines, center), and 52% of HCC patients with upregulation of *ADAMTSL5* mRNA levels (black, right). Bottom: graph reporting the three subgroup of HCC patients according to low, unchanged, and high *ADAMTSL5* expression levels (numbers and percentages are indicated). Data correspond to RNA-seq studies of a cohort of 371 HCC patients (from TCGA). Note that *ADAMTSL5* is overexpressed in 193 out of 371 HCC patients (52%; Log₂FC>1; FDR<0.05). It is concluded that high *ADAMTSL5* transcript levels can be detected in more than 50% of HCC patients.

In Fig.7(B), the table reports the presence (black line) or the absence of major HCC risk factors in all 371 analyzed patients. Note that HCC patients with increased *ADAMTSL5* levels are significantly associated, although not exclusively, to alcohol taken. It is concluded that high mRNA levels of *ADAMTSL5* is present in a large proportion of patients characterized by several risk factors, with a predominance for those associated with alcohol taken.

Example 8: ADAMTSL5 protein levels are overexpressed in a vast majority of HCC analyzed patients.

The figure reports immunostaining with anti-ADAMTSL5 antibodies in tumor samples compared to the adjacent liver. Note strong ADAMTSL5 protein levels (dark staining; by Fast Red) in all analyzed HCC patients (except #8 patient) . It is concluded that high ADAMTSL5 protein levels characterize a vast majority of HCC patients.

Example 9: ADAMTSL5 overexpression in a panel of human cancer cell lines.

The graph shows the mRNA expression levels of *ADAMTSL5* in a panel of human HCC cell lines, in MKN (gastric cancer) cell line, in human breast cell lines, in HELA (human cervix) cancer cell line, and in HEK (human embryonic kidney) cell line. Mean value of three independent experiments. It is concluded that high mRNA levels of *ADAMTSL5* are present in cancer cells with different origin.

## Claims

1. A method for diagnosing an hepatocellular carcinoma in a mammal in a need thereof, comprising the following steps:
providing a biological sample collected from said mammal;
determining, from said biological sample, if the ADAMTSL5 gene is overexpressed;
diagnosing an hepatocellular carcinoma from the determination of the overexpression of said gene.

2. The method according to claim 1, wherein the biological sample is selected from the group consisting of blood, biopsy tissue, blood serum, blood plasma, urine, stool, sputum, cerebrospinal fluid, or supernatant from cell lysate, preferably the biological sample is tissue biopsy, blood, blood plasma, blood serum, or urine.

3. The method according to any one of the preceding claims, wherein the overexpression of ADAMTSL5 gene in the biological sample is determined by measuring the ADAMTSL5 protein levels or mRNA levels in said biological sample.

4. The method according to claim 3, wherein the ADAMTSL5 protein levels in the biological sample are measured by adding at least one antibody anti-ADAMTSL5 type to said biological sample.

5. The method according to claim 4, wherein the antibody anti-ADAMTSL5 type is selected from the group consisting of uncoupled or coupled with alkaline phosphatase horse-radish peroxidase, or with fluorescent dyes.

6. The method according to any one of the claims 3 to 5, wherein the ADAMTSL5 protein levels in the biological sample are measured by using immunostaining, immunofluorescence, western blot, or ELISA.

7. The method according to claim 3, wherein the *ADAMTSL5* mRNA levels in the biological sample are measured by available methodologies to detect mRNA to said biological sample, including microarray, RNA-seq, in situ hybridization, RNA-scope, as well as regular, semi-quantitative, or quantitative RT-PCRs.

8. A pharmaceutical composition comprising an agent targeting ADAMTSL5 and a pharmaceutically acceptable carrier for use in the treatment of an hepatocellular carcinoma, wherein the agents targeting ADAMTSL5 are selected from the group consisting of blocking antibodies, sh-RNA, si-RNA, and antisense RNA against ADAMTSL5.

9. An *in vitro* use of an ADAMTSL5 protein as a biomarker of an hepatocellular carcinoma.

10. An *in vitro* method for monitoring the response to an anticancer treatment of a mammal suffering from hepatocellular carcinoma comprising determining the ADAMTSL5 level of expression in a biological sample of said mammal at two or more time points during said anticancer treatment, wherein an equal or higher ADAMTSL5 level of expression in a biological sample of the subject at a later time point, compared to a reference value obtained in a biological sample of the subject at an earlier time point, is indicative of a resistance of the subject to said anticancer treatment whereas a lower ADAMTSL5 level is indicative of a response of the subject to said anticancer treatment.

## Patentansprüche

1. Verfahren zum Diagnostizieren eines hepatozellulären Karzinoms bei einem Säugetier, das dessen bedarf, die folgenden Schritte umfassend:
Bereitstellen einer biologischen Probe, die von dem Säugetier gesammelt wurde;
Bestimmen anhand der biologischen Probe, ob das ADAMTSL5-Gen überexprimiert wird,
Diagnostizieren eines hepatozellulären Karzinoms anhand der Bestimmung der Überexpression des Gens.

2. Verfahren nach Anspruch 1, wobei die biologische Probe ausgewählt ist aus der Gruppe bestehend aus Blut, Biopsiegewebe, Blutserum, Blutplasma, Urin, Stuhl, Sputum, Zerebrospinalflüssigkeit, oder Überstand aus Zelllysat, wobei es sich vorzugsweise bei der biologischen Probe um Gewebebiopsie, Blut, Blutplasma, Blutserum, oder Urin handelt.

3. Verfahren nach einem der vorstehenden Ansprüche, wobei die Überexpression des ADAMTSL5-Gens in der biologischen Probe durch Messen der ADAMTSL5-Proteinspiegel oder mRNA-Spiegel in der biologischen Probe bestimmt wird.

4. Verfahren nach Anspruch 3, wobei die ADAMTSL5-Proteinspiegel in der biologischen Probe durch Zugeben von mindestens einem Antikörper vom Anti-ADAMTSL5-Typ zu der biologischen Probe gemessen werden.

5. Verfahren nach Anspruch 4, wobei der Antikörper vom Anti-ADAMTSL5-Typ ausgewählt ist aus der Gruppe bestehend aus nicht gekoppelt oder mit alkalischer Phosphatase Meerrettichperoxidase, oder mit Fluoreszenzfarbstoffen gekoppelt.

6. Verfahren nach einem der Ansprüche 3 bis 5, wobei die ADAMTSL5-Proteinspiegel in der biologischen Probe unter Verwendung von Immunfärbung, Immunfluoreszenz, Western Blot, oder ELISA gemessen werden.

7. Verfahren nach Anspruch 3, wobei die *ADAMTSL5-mRNA-*Spiegel in der biologischen Probe mit verfügbaren Methoden, um mRNA in der biologischen Probe nachzuweisen, einschließlich Microarray, RNA-Seq, In-situ-Hybridisierung, RNA-Scope, sowie regulären, halbquantitativen oder quantitativen RT-PCRs gemessen werden.

8. Pharmazeutische Zusammensetzung, die ein auf ADAMTSL5 zielendes Mittel und einen pharmazeutisch akzeptablen Träger umfasst, zur Verwendung in der Behandlung eines hepatozellulären Karzinoms, wobei die auf ADAMTSL5 zielenden Mittel ausgewählt sind aus der Gruppe bestehend aus blockierenden Antikörpern, sh-RNA, si-RNA, und Antisense-RNA gegen ADAMTSL5.

9. *In-vitro*-Verwendung eines ADAMTSL5-Proteins als Biomarker für ein hepatozelluläres Karzinom.

10. In-vitro-Verfahren zur Überwachung des Ansprechens eines an einem hepatozellulären Karzinom leidenden Säugetieres auf eine Krebsbehandlung, welches das Bestimmen des ADAMTSL5-Expressionsspiegels in einer biologischen Probe des Säugetieres zu zwei oder mehr Zeitpunkten während der Krebsbehandlung umfasst, wobei ein gleicher oder höherer ADAMTSL5-Expressionsspiegel in einer biologischen Probe des Subjekts zu einem späteren Zeitpunkt, verglichen mit einem Referenzwert, der in einer biologischen Probe des Subjekts zu einem früheren Zeitpunkt erhalten wurde, eine Resistenz des Subjekts gegenüber der Krebsbehandlung anzeigt, wohingegen ein niedrigerer ADAMTSL5-Spiegel ein Ansprechen des Subjekts auf die Krebsbehandlung anzeigt.

## Revendications

1. Procédé de diagnostic d'un carcinome hépatocellulaire chez un mammifère en ayant besoin, comprenant les étapes suivantes :
la fourniture d'un échantillon biologique prélevé sur ledit mammifère ;
la détermination, à partir dudit échantillon biologique, si le gène ADAMTSL5 est surexprimé ;
le diagnostic d'un carcinome hépatocellulaire à partir de la détermination de la surexpression dudit gène.

2. Procédé selon la revendication 1, dans lequel l'échantillon biologique est sélectionné dans le groupe constitué du sang, d'un tissu de biopsie, du sérum sanguin, du plasma sanguin, de l'urine, des selles, des crachats, du liquide céphalo-rachidien, ou d'un surnageant de lysat cellulaire, de préférence l'échantillon biologique est une biopsie de tissu, du sang, du plasma sanguin, du sérum sanguin, ou de l'urine.

3. Procédé selon l'une quelconque des revendications précédentes, dans lequel la surexpression du gène ADAMTSL5 dans l'échantillon biologique est déterminée en mesurant les taux de protéine ADAMTSL5 ou les taux d'ARNm dans ledit échantillon biologique.

4. Procédé selon la revendication 3, dans lequel les taux de protéine ADAMTSL5 dans l'échantillon biologique sont mesurés en ajoutant au moins un anticorps anti-ADAMTSL5 audit échantillon biologique.

5. Procédé selon la revendication 4, dans lequel l'anticorps anti-ADAMTSL5 est sélectionné dans le groupe constitué de non couplé ou couplé à la phosphatase alcaline, à la peroxydase de raifort, ou à des colorants fluorescents.

6. Procédé selon l'une quelconque des revendications 3 à 5, dans lequel les taux de protéine ADAMTSL5 dans l'échantillon biologique sont mesurés par immunocoloration, immunofluorescence, transfert de western, ou ELISA.

7. Procédé selon la revendication 3, dans lequel les taux d'ARNm *d'ADAMTSL5* dans l'échantillon biologique sont mesurés par les méthodologies disponibles pour détecter un ARNm dans ledit échantillon biologique, comportant un microréseau, un séquençage d'ARN, une hybridation in situ, un RNA-scope, ainsi que les RT-PCR ordinaires, semiquantitatives, ou quantitatives.

8. Composition pharmaceutique comprenant un agent ciblant ADAMTSL5 et un support pharmaceutiquement acceptable pour une utilisation dans le traitement d'un carcinome hépatocellulaire, dans laquelle les agents ciblant ADAMTSL5 sont sélectionnés dans le groupe constitué des anticorps bloquants, d'un ARN-sh, d'un ARN-si, et d'un ARN antisens dirigé contre ADAMTSL5.

9. Utilisation *in vitro* d'une protéine ADAMTSL5 comme biomarqueur d'un carcinome hépatocellulaire.

10. Procédé *in vitro* pour surveiller la réponse à un traitement anticancéreux d'un mammifère souffrant d'un carcinome hépatocellulaire comprenant la détermination du niveau d'expression d'ADAMTSL5 dans un échantillon biologique dudit mammifère à deux moments temporels ou plus au cours dudit traitement anticancéreux, dans lequel un niveau d'expression d'ADAMTSL5 égal ou plus élevé dans un échantillon biologique du sujet à un moment temporel ultérieur, par rapport à une valeur de référence obtenue dans un échantillon biologique du sujet à un moment temporel antérieur, indique une résistance du sujet audit traitement anticancéreux tandis qu'un taux d'ADAMTSL5 plus bas indique une réponse du sujet audit traitement anticancéreux.
